# EUROPEAN PATENT SPECIFICATION

(11) **EP 3 352 592 B1**
(45) Date of publication and mention of the grant of the patent: **31.07.2019**
(21) Application number: 16766069.5
(22) Date of filing: 12.09.2016
(51) Int. Cl.: A24F 47/00, A61M 11/04, A61M 15/06

(54) **AEROSOL PROVISION SYSTEM AND BATTERY SECTION THEREFOR**
AEROSOLBEREITSTELLUNGSSYSTEM UND BATTERIEABSCHNITT DAFÜR
SYSTÈME DE PRODUCTION D'AÉROSOL ET SECTION DE BATTÉRIE POUR LEDIT SYSTÈME

(30) Priority: 22.09.2015 GB 201516792
(43) Date of publication of application: 01.08.2018
(73) Proprietor: Nicoventures Holdings Limited, London WC2R 3LA (GB)
(72) Inventor: LEADLEY, David, London WC2R 3LA (GB); HEPWORTH, Richard, London WC2R 3LA (GB); TRAN, My-Linh, London WC2R 3LA (GB)
(74) Representative: D Young & Co LLP
(86) International application number: PCT/GB2016/052808
(87) International publication number: WO 2017/051150

(56) References cited:
- EP-A1- 2 724 741
- WO-A1-2016/028544
- CN-U- 204 180 932
- CN-U- 204 273 239
- CN-U- 204 466 915
- US-A1- 2013 167 854
- US-A1- 2014 261 408
- US-A1- 2014 286 630
- US-A1- 2015 080 053

## Description

### Technical Field

The present invention relates to an aerosol or vapour provision system with an air inlet.

### Background

Aerosol provision systems such as e-cigarettes generally contain a reservoir of a source liquid containing a formulation, typically including nicotine, from which an aerosol is generated, such as through vaporisation or other means. Thus an aerosol source for an aerosol provision system may comprise a heating element coupled to a portion of the source liquid from the reservoir. When the heating element is activated it causes vaporisation of a small amount of the source liquid, which is thus converted to an aerosol for inhalation by the user. More particularly, such devices are usually provided with one or more air inlet holes which may or may not be located away from a mouthpiece of the system. When a user sucks on the mouthpiece, air is drawn through the inlet holes and past the aerosol source. There is an air flow path connecting the inlet holes to the aerosol source and on to an opening in the mouthpiece so that air drawn past the aerosol source continues along the flow path to the mouthpiece opening, carrying some of the aerosol from the aerosol source with it. The aerosol-carrying air exits the aerosol provision system through the mouthpiece opening for inhalation by the user.

Some aerosol provision systems are configured in two sections. An aerosol provision section houses the reservoir of source liquid and one or more heating elements, and has the airflow path defined therethrough from the inlet hole(s) to the mouthpiece. A battery section houses a battery (which may be replicable or rechargable) for providing electrical power to the heating element. An electrical connection is provided between the two sections. The sections can be separable from one another, in which case there is also a mechanical connection between the sections; this typically also makes the electrical connection.

The two sections can be arranged linearly so that the battery section is connected at the opposite end of the aerosol provision section to the mouthpiece. This gives a generally elongate device in which the battery is aligned substantially along the direction of airflow in the flow path, and when the mouthpiece points upwards, as it does in use, the battery section is underneath the aerosol provision section. The air inlet can be located in a side wall of the aerosol delivery section just below the base of the reservoir, that is, the part of the reservoir remote from the mouthpiece. This gives a short and simple air flow path to the heating element (which is generally in or near the reservoir). However, there is a risk that the user will cover this air inlet with his hand or fingers when using the device. As well as inhibiting or reducing aerosol delivery, this can be unsafe in devices in which the heating element is switch-operated under control of the user. A lack of airflow can then lead to overheating.

CN 204466915 U, CN 204180932 U, US 2013/0167854 A1 and EP 2724741 A1 describe various electronic cigarettes in which a section that includes a battery has a surface facing a surface of a section for delivering aerosol, where air can be taken into the aerosol delivery section. The surfaces are located inside exterior housings. US 2015/0080053 A1 describes a cellular device case having at least one edge to allow the mounting of a cellular device therein, a recess to house a battery, a voltage regulator to vary the output of a battery, and a power connector to allow the connection of an electronic vapor device.

### Summary

According to the invention, there is provided a vapour provision system according to claim 1, and a battery section according to claim 11. The dependent claims define preferred embodiments of the invention.

According to a first aspect of the invention, there is provided a vapour provision system comprising: an aerosol delivery section configured to generate aerosol from liquid in a reservoir; an airflow path through the aerosol delivery section extending from an air inlet to a mouthpiece; a battery section configured to join to the aerosol delivery section and house a battery to provide electrical power to one or more components in the aerosol delivery section, the battery section arranged laterally to at least a portion of the aerosol delivery section with respect to a direction of airflow through the mouthpiece; an interface region in which a surface of the aerosol delivery section faces a surface of the battery section when the sections are joined; wherein the air inlet is located on the aerosol delivery section in the interface region so as to take in air that has been channelled over part of the battery section; and the surface of the battery section in the interface region comprises at least one groove with at least one end located at an edge of the interface region, such that, when the battery section and the aerosol delivery section are joined: a first cavity is at least partially formed in the interface region by the at least one groove, the first cavity having at least one external opening to air defined by the at least one end of the at least one groove; and the at least one external opening to air is in airflow communication with the air inlet via said first cavity.

The battery section may be arranged laterally to the aerosol delivery section with respect to a direction of airflow through the mouthpiece.

The battery section may have a connecting portion that extends laterally to receive a base part of the aerosol delivery section, the air inlet being located in a base wall of the aerosol delivery section that faces the connecting portion when the sections are joined, wherein a surface of the connecting portion that faces the base part of the aerosol delivery section has formed therein the at least one groove.

The at least one groove may extend radially with respect to a central axis of the aerosol delivery section when joined to the battery section to said at least one end of the at least one groove.

The base part of the aerosol provision section and the said surface of the connecting portion may be shaped to form a central cavity in the interface region with which each groove is in airflow communication at an end opposite to the external opening end, the air inlet being in airflow communication with the central cavity.

The central cavity may house an electrical connection between the battery section and the aerosol delivery section.

Said surface of the battery section in the interface region may be a side wall of the battery section and the said surface of the aerosol delivery section in the interface region may be a side wall of the aerosol delivery section.

The at least one groove may extend across the said side wall of the battery section from a first end to a second end, each of the first and second ends forming one of said external openings to air. Alternatively, each groove may have an end forming one of said external openings to air, said end located at an edge of the interface proximate the mouthpiece.

The first cavity may have at least two external openings to air.

The vapour provision system may further comprise an adjustable element configured to enable an effective size of the air inlet to the altered, so as to vary the level of airflow along the airflow path.

In a comparative example not falling within the scope of the invention, there is described is an aerosol delivery section for a vapour provision system which is configured to generate aerosol from liquid in a reservoir when joined to a battery section housing a battery, the aerosol delivery section comprising: an air inlet; an airflow path through the aerosol delivery section from the air inlet to a mouthpiece; and an interface region comprising a surface of the aerosol delivery section configured to face a surface of a battery section when the aerosol delivery section is joined to said battery section; wherein the air inlet is located in the interface region so as to take in air that has been channelled over part of the battery section when the sections are joined. The air inlet may be concealed from a user when the aerosol delivery section is joined to a battery section. In some examples, the air inlet may be located in a base wall or a side wall of the aerosol delivery section.

According to a second aspect of the invention, there is provided a battery section for a vapour provision system, the battery section configured to join to an aerosol delivery section of the vapour provision system, the aerosol delivery section configured to generate aerosol from liquid in a reservoir, the vapour provision system further comprising an airflow path through the aerosol delivery section extending from an air inlet to a mouthpiece, the battery section configured to house a battery to provide electrical power to said aerosol delivery section when the battery section is joined to said aerosol delivery section, the battery section configured to be arranged laterally to at least a portion of the aerosol delivery section with respect to a direction of airflow through the mouthpiece, the battery section comprising: an interface region comprising a surface of the battery section configured to face a surface of said aerosol delivery section when the battery section is joined to said aerosol delivery section; and at least one groove formed in said surface of the battery section in the interface region positioned for alignment with said air inlet in said aerosol delivery section, the at least one groove having at least one end at an edge of the interface region, such that, when the battery section and the aerosol delivery section are joined: a first cavity is at least partially formed in the interface region by the at least one groove, the first cavity having at least one external opening to air defined by the at least one end of the at least one groove; and the at least one external opening to air is in airflow communication with the air inlet via said first cavity. The battery section may have a base portion that extends laterally to which an aerosol delivery section may be joined.

It will be appreciated that features of the dependent claims may be combined with each other and features of the independent claims in combinations other than those explicitly set out in the claims, as long as the resulting subject-matter falls within the scope of one of the independent claims.

### Brief Description of the Drawings

Various embodiments of the invention and comparative examples not falling within the scope of the invention will now be described in detail by way of example only with reference to the accompanying drawings in which:
Figure 1 shows a schematic side view representation of an electronic cigarette to which embodiments of the invention are applicable;
Figure 1A shows an enlarged view of a lower part of the electronic cigarette of Figure 1;
Figures 2A and 2B show simplified side and top views of an electronic cigarette with regions of particular interest to the present invention highlighted;
Figure 3 shows a schematic cross-sectional view of an aerosol delivery section of an electronic cigarette in accordance with a comparative example;
Figure 4 shows a schematic cross-sectional view of a further aerosol delivery section coupled to a battery section in accordance with a comparative example;
Figures 5A, 5B and 5C show an enlarged partial cross-sectional view, a plan view and a side view of parts of an electronic cigarette in accordance with an embodiment of the invention;
Figures 6A and 6B show an enlarged partial cross-sectional view and a plan view of parts of an electronic cigarette in accordance with another embodiment of the invention;
Figures 7A and 7B show an enlarged partial cross-sectional view and a plan view of parts of an electronic cigarette in accordance with a further embodiment of the invention;
Figure 8 shows a perspective view of a battery section of an electronic cigarette;
Figures 9A and 9B show side views of a battery section according to an embodiment of the invention, and an aerosol delivery section suitable for use with said battery section, respectively;
Figures 10, 11 and 12 show side views of three example battery sections configured in accordance with further embodiments of the invention;
Figure 13A and 13B show a side view and a top view of a battery section in accordance with an alternative embodiment of the invention;
Figure 14 shows a side view of battery section in accordance with a different embodiment of the invention;
Figure 15 shows a cross-sectional side view of an electronic cigarette in accordance with a yet further embodiment of the invention; and
Figure 16 shows a schematic side view of an aerosol delivery section according to various comparative examples.

### Detailed Description

Aspects and features of certain embodiments of the invention and of comparative examples not falling within the scope of the invention are discussed/ described herein. Some aspects and features of certain examples and embodiments may be implemented conventionally and these are not discussed/described in detail in the interests of brevity. It will thus be appreciated that aspects and features of apparatus and methods discussed herein which are not described in detail may be implemented in accordance with any conventional techniques for implementing such aspects and features.

As described above, the present disclosure relates to aerosol provision systems, such as e-cigarettes. Throughout the following description the terms "e-cigarette" or "electronic cigarette" may sometimes be used; however, it will be appreciated these terms may be used interchangeably with aerosol (vapour) provision system.

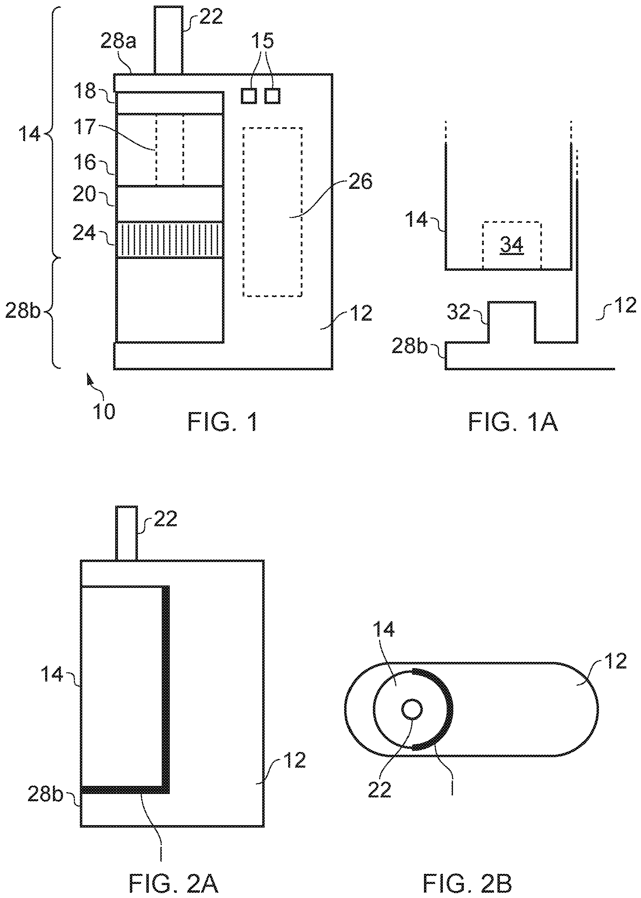

Directional terms in the present application, such as upper, lower, top, bottom, side and the like, are not to be considered limiting and are used for convenience and brevity, and consistency with the Figures. The terms apply to the typical orientation of an e-cigarette in use, when the mouthpiece points upward (such as in Figure 1), and should be interpreted accordingly if considering a different orientation of an e-cigarette.

Figure 1 is a schematic diagram of an example aerosol/vapour provision system such as an e-cigarette 10 to which some embodiments are applicable. The e-cigarette 10 is a modular device comprising a battery section 12 and an aerosol delivery section 14 which are mechanically and electrically connected together. The battery section 12 houses a battery 26 and has one or more buttons or switches 15 for a user to operate to deliver electrical power to one or more components in the aerosol delivery section 14. The battery section 12, in this example, has two supporting sections 28a, 28b which extend laterally to receive and support the aerosol delivery section 14. In particular, the lower supporting section 28b is a connecting portion which makes the necessary electrical connections to the battery 26, and also provides a mechanical connection which may be, for example, a screw thread connection between cooperating screw threads on the connection portion 28b and the base of the aerosol delivery section 14, or a friction "push fit" style of connection. The aerosol delivery section 14 is cylindrical in this example, and at its base has a grip portion 24 provided with vertical grooves or other surface features to facilitate grip, so that a user can hold this part and rotate the aerosol delivery section 14 to disengage the screw threads, or pull to overcome the friction fit. Alternatively the two sections may be shaped to slot or fit together, with a latch, clip, hinged portion or other retaining element provided to secure the two section together in the joined configuration. Other joining arrangements may also be used. Once the relevant parts are disengaged or separated, the aerosol delivery section 14 can be lifted free from the battery section 12.

Figure 1A shows a simplified schematic partial side view of the lower part of the system 10 according to an example. The aerosol delivery section 14 is shown separated from the battery section 12. The battery section has an upwardly protruding connector 32 extending from the upper face of the connecting portion 28b, where the upper face is a wall or surface that faces the aerosol delivery section when the two sections are joined. The connector 32 is an electrode in a suitable housing or cover, by which an electrical connection can be made between the battery in the battery section 12 and components such as a heating element in the aerosol delivery section 14. The aerosol delivery section has a corresponding socket 34 formed inwardly on its lower face or wall which fits over the connector 32 and houses one or more electrical contacts which are brought into contact with the electrode to form the electrical connection when the sections are joined together.

The aerosol delivery section 14 comprises the aforementioned grip section 24 at its base, above which is a tank base section 20. A tank or reservoir 16 extends upwardly from this base section 20 and is formed by transparent walls so that a user can conveniently observe liquid solution contained in the tank 16. The walls need not be transparent, however. A tube or pipe 17 runs centrally up through the tank 16; this defines part of the airflow path that runs through the aerosol delivery section 14. Disposed within the pipe 17 are one or more wicks mounted within or around one or more heating elements that might be in the form of coils (not shown). The wicks absorb liquid from the tank, the heating coils are heated when electrical current is supplied to them from the battery 26, and the liquid in the wicks is vaporised, and carried away on air flowing through the pipe 17. A lid 18 is provided to close the upper end of the tank 16. The lid 18 can be removed to allow the tank 16 to be refilled when the aerosol delivery section 14 is disconnected from the battery section 12. The airflow path passes through the lid 18 to a mouthpiece 22 through which a user can inhale to generate the required airflow along the airflow path. The mouthpiece, also known as a "drip tip", may or may not be removable and / or replaceable.

The opposite end of the air flow path within the aerosol delivery section 14 to the mouthpiece 22 is defined by at least one air inlet (not shown in Figure 1) in the outer surface of the aerosol delivery section 14 which connects to the pipe 17. When a user inhales through the mouthpiece 22, air is taken in through the air inlet to flow along the air flow path.

Locating the air inlet in an area such as the exposed side of the tank base section 20 provides a conveniently short path to the heating coils and wick. However, an inlet in this location is vulnerable to being wholly or partially covered by the user's hands or fingers as they hold the e-cigarette to inhale through it.

Embodiments of the present invention propose positioning the air inlet to address this issue. In particular, the air inlet can be positioned so that air reaching it is not drawn directly in through a wall of aerosol delivery section 14, but instead is caused to flow over a part of the battery section 12 before reaching the air inlet. This can be achieved by locating the air inlet in an interface region where part of the aerosol delivery section faces part of the battery section, and shaping and configuring parts of the battery section and/or the aerosol delivery section in this interface region to facilitate this air flow by providing air flow communication between the air inlet and an external opening to air formed at the edge of the interface region where the aerosol delivery section is adjacent to the battery section. Arrangements in accordance with this proposal can configure this external open end of the air flow path so that the risk of the path being blocked when holding the device is reduced or avoided.

Figures 2A and 2B are highly schematic representations of an e-cigarette 10 having a battery section 12 and an aerosol delivery section 14, to illustrate example positions of the interface region. The sections may be differently shaped than shown, with a differently shaped interface; these representations are examples only. The interface region is considered to be any area in which an outer surface or wall of the aerosol delivery section 14 faces an outer surface or wall of the battery section 12 when the two sections are joined together. In the side view of Figure 2A, an interface region of interest is shown by the heavy line I, and comprises the area in which the base part of the aerosol delivery section 14 is received on the upper face of the connecting portion 28b, and the area in which a side wall of the aerosol delivery section 14 faces an adjacent side wall of the battery section 12. Note that the interface region extends through the thickness of the e-cigarette over the whole extent of the facing surfaces, and is not limited to the edges only. Figure 2B shows a plan view of the system 10 from above, from which it can be seen that the interface region I between the side walls of the sections 12, 14 extends up to the top surface of the e-cigarette 10. In accordance with embodiments of the invention, the air inlet for the airflow channel is located on a surface of the aerosol delivery section 14 that is within the interface region, and at least one external opening to air that delivers air to the air inlet is located at an edge of the interface region, such as is indicated by the lines I in Figure 2A and 2B.

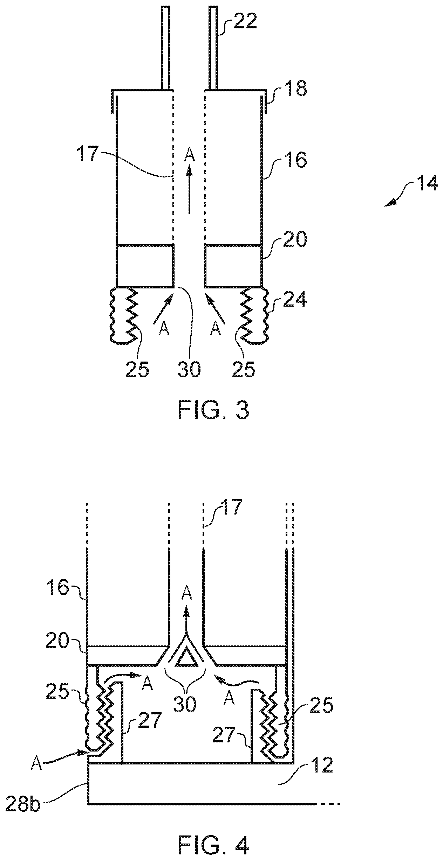

Figure 3 shows a cross-sectional schematic view of an aerosol delivery section 14 configured according to some comparative examples. In this example, the grip section 24 has a screw thread 25 on its inner surface for coupling with a corresponding thread on the battery section 12. Above this lies the tank base section 20, which in this example forms a base wall of the aerosol delivery section 14. Other configurations may be envisaged where parts other than the tank base form the base wall, for example if other components are installed below the tank 16, or if a connecting mechanism other than a screw thread is employed, such as a push or snap fit. The base wall faces the upper face of the connecting portion 28b of a battery section when a battery section is joined to the aerosol delivery section, giving an interface region. Regardless of the nature of the base wall, in this example the air inlet 30 is formed in this base wall, and connects with the pipe 17 so that air taken in through the inlet 30 flows along the flow path and into the pipe 17 to the heating coil or element (not shown) and subsequently out through the mouth piece 22, as indicated by the arrows A. The air inlet 30 need not be centrally located as illustrated, and may also comprise two or more separate inlets which each connect with the pipe 17. The term "air inlet" is intended to cover arrangements with a single inlet and also with more than one inlet where these inlets each supply air to the airflow path.

To enable air intake into the air inlet 30 when the aerosol delivery section 14 is joined to its battery section, embodiments of the invention and comparative examples propose various configurations for one or more external aperture or openings to air displaced from the air inlet 30 and located at the interface edge, but in airflow communication with the air inlet 30. This allows the initial air entry point for the airflow path to be situated so that the risk of accidental blockage is mitigated. In some embodiments and examples, this is achieved by moving this initial intake to a relatively low position on the e-cigarette so that the risk of a user blocking the air flow with his hand is reduced.

Figure 4 shows a cross-sectional schematic view of a vapour provision system in accordance with such a comparative example. The aerosol delivery section 14 is largely as in Figure 3, except that the air inlet 30 in this example comprises a plurality of individual inlets in an annular arrangement that connect by channels to the pipe 17 (or the air inlet might be a single annular aperture). The aerosol delivery section 14 is shown coupled to the battery section 12 via the screw thread 25 which is engaged with a corresponding screw thread 27 upstanding from the upper face of the connecting portion 28b of the battery section 12. Note that for simplicity the electrical connection is not shown. The screw threads are configured such that when they are fully tightened together there are one or more gaps extending through the threads that form an air flow pathway or channel by which air can enter from the external environment, traverse the screw thread into the volume of the interface region (under the base wall 20 of the tank 16) and so through the air inlet 30 to the pipe 17, as shown by the arrows A. This might be achieved by having breaks in the screw thread, or otherwise forming the screw threads so that when coupled they form a seal which is leaky or not airtight. By using the screw thread in this way, the external opening to air may be caused to extend around a large part of the lower edge (circumference) of the aerosol delivery section 14 so that it is very unlikely that a user would completely cover the air intake in use.

In other comparative examples, alternative engagement arrangements between the aerosol delivery section 14 and the battery section can be configured to enable air intake in a similar manner to a leaky screw thread. For example, the aerosol delivery section and the battery section may be provided with cooperating engaging elements that are shaped to provide a friction fit when the two sections are pushed together or to provide a mechanical attachment by means of protruding lugs, a collar or similar element on one section that fit over or into depressions or hollows in the other section when the two sections are pushed together. These elements can be shaped so that when the sections are coupled or joined the resulting attachment is not airtight, and allows air to pass through to reach the air inlet in the base of the aerosol delivery section.

Other arrangements are possible in which the air inlet is located in the base wall of the aerosol delivery section, the interface region being between the aerosol delivery section base and the upper surface of the connecting portion.

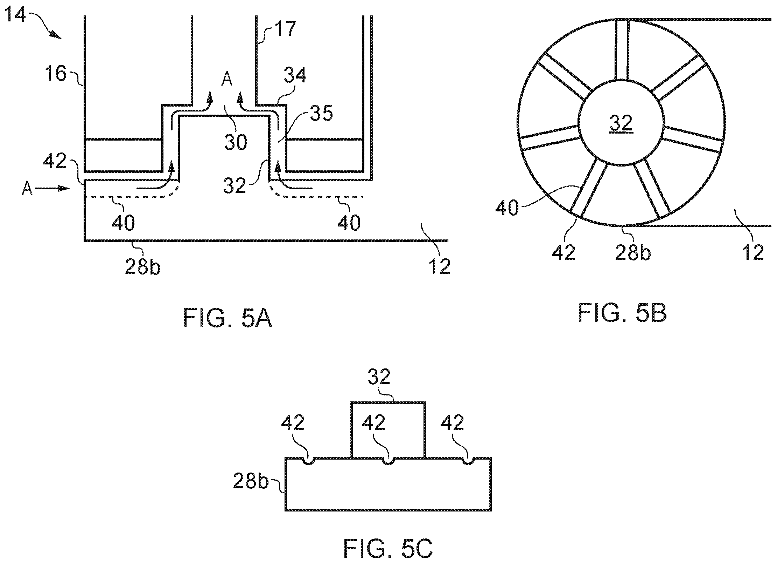

Figure 5A shows a cross-sectional view through the interface region of an embodiment of the invention having a base wall air inlet. The aerosol delivery section 14 is joined to the connecting portion 28b of the battery section 16. It has a socket 34 which engages with a connector 32 on the connecting portion 28b (as in Figure 1A) to make the required electrical connection (not shown). A central air inlet 30 is formed in the base wall, within the socket 34. The socket is shaped so as to be larger than the connector so that there is a cavity 35 between the walls of the socket 34 and the walls of the connector 32, which is in airflow communication with the air inlet 30. To enable air to enter this cavity and thence the air inlet 30, the upper face of the connecting portion 28b of the battery section 16 has formed in it a number of grooves 40. The grooves have one end at or near to the connector 32 so that this end opens into the cavity between the connector 32 and the socket 34. In this embodiment, the aerosol delivery section 14 has the same outer profile as the connecting section 28b at the interface region edge, so the grooves 40 have opposite ends in the side walls of the connecting section 28b, to form an external opening to air 42 at the end of each groove 40. Thus, when the aerosol delivery section is connected, air can enter the external openings, flow along the grooves 40 (under the base wall of the aerosol delivery section 14 and over those parts of the surface of the connecting portion that form the grooves) to the cavity and into the air inlet 30.

Figure 5B shows a plan view (to a different scale) of the connecting portion 28b, extending laterally from the battery section 16. In this example, there are seven grooves 40 arranged radially so that they extend inwardly from the ends in the side walls of the connecting portion towards the central connector 32. In effect, they are radially arranged with respect to a central longitudinal axis of the aerosol delivery section 14 when it is joined to the battery section 16. However, other arrangements are possible, for example if the connector and socket are not centrally disposed, or if a different configuration is otherwise preferred. For example, there may be a plurality of parallel grooves arranged with one end at the outer edge and one end reaching to the cavity. Groups of parallel grooves might be spaced apart around the connecting portion. Any number of grooves, from one upwards, can be used as required to achieve a desired level of air intake and airflow in the airflow path. The grooves need not be straight, and they may have a width that changes along their length. However, a larger number of grooves gives a larger number of external openings to air, which decreases the risk of the air intake being blocked by a user holding the e-cigarette. The grooves together can be thought of as comprising part of the cavity 35, so that the outer ends 42 of the grooves or recesses are the external openings to air of the cavity.

Figure 5C shows a side view (to a different scale) of the connecting portion 28b, depicting how the grooves extend out to the side walls of the connecting portion 28b so that their ends 42 appear as notches.

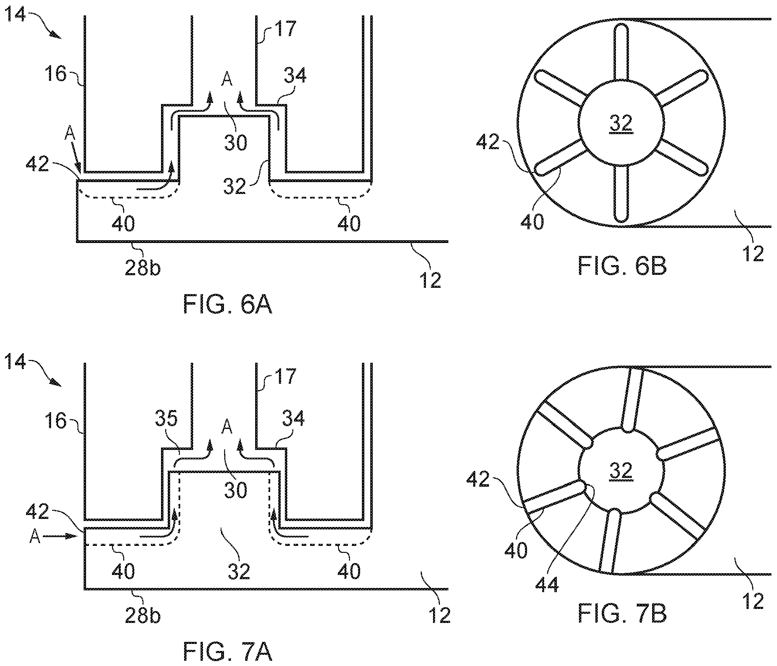

Figure 6A shows an alternative embodiment of the invention comprising grooves in the connecting portion upper face. Many features are the same as in the embodiment of Figures 5A to 5C, and the remarks made regarding that embodiment apply also to this embodiment. A difference from the Figure 5A embodiment is that the aerosol delivery section 14 has a smaller width (diameter in this cylindrical example) than the connecting portion 28b on which it sits, so that the edges of the connecting portion 28b extend beyond the base of the aerosol delivery section 14. This means that there is no need for the grooves to extend out into the side walls of the connecting portion 28b. Instead, they terminate in the upper face of the connecting portion 28b at a point beyond the outer edge of the aerosol delivery section, to form the external openings to air 42.

Figure 6B shows a corresponding plan view (to a different scale) of the connecting portion 28b, in which it can be seen that the grooves (six in this embodiment) are again radially arranged, but have outer ends located inwardly from the edge (side wall) of the connecting portion 16.

Figure 7A shows a further alternative embodiment of the invention comprising grooves in the connecting portion upper face. Again, many features are the same as in the embodiment of Figures 5A to 5C, and the remarks made regarding that embodiment apply also to this embodiment. A difference from the Figure 5A embodiment is that the socket 34 and the connector 32 have closely matched diameters (or other width dimensions if not circular) so that the cavity 35 is limited to only a space above the connector 32 and below the air inlet 30 in the base of the socket 34. To enable air to reach the cavity 35 to enter the air inlet 30, the grooves 40, again in the upper face of the connecting portion, extend also up the sides of the connector 32 and terminate in the upper face of the connector 32 to communicate with the cavity 35. The outer ends 42 of the grooves 40 defining the external openings to air are formed in the outer wall of the connecting portion 28b, as in Figure 5A, but may instead be formed as in Figure 6A.

Figure 7B shows a corresponding plan view (to a different scale) of the connecting portion 28b, in which it can be seen that the grooves (five in this embodiment) are again radially arranged, have outer ends 42 in the side wall of the connecting portion 16, and inner ends 44 in the upper face of the connector 32 to give airflow communication into the cavity 35.

In other embodiments, the air inlet can be located in the side wall of the aerosol delivery section, so as to take in air received via the interface region between the side wall of the aerosol delivery section and the facing side wall of the battery section. This arrangement moves the air inlet away from electrical connection. This can be beneficial in reducing the risk of any leakage of the source liquid from the tank to the electrical contacts via the airflow pathway.

To achieve such an arrangement, the outer surface of the battery section can be shaped so as to include one or more grooves in the wall that faces and abuts the aerosol delivery section. The grooves extend out to the edge of the interface region. When the sections are placed together, this shaping defines one or more cavities (multiple cavities are considered still as one cavity for understanding of these embodiments) in the interface region, which have an external communication to air via ends of the grooves that reach to the edge of the interface region and form openings thereat. The air inlet (which might be one or more individual apertures that connect with the pipe 17 as described with regard to Figures 3 and 4) is created in the aerosol delivery section in a position that is aligned with the cavity. Thus, air can be taken into the airflow path by entering through the external opening(s) into the cavity and then into the air inlet.

The aerosol delivery section and the battery section can be configured to ensure that the alignment of the air inlet with the cavity is achieved and maintained when the two sections are joined. For example, there may be shaped co-operating parts that allow the sections to be engaged only in the required orientation, or a screw thread coupling may be structured to that the air inlet and the cavity are brought into alignment when the screw thread is fully fastened.

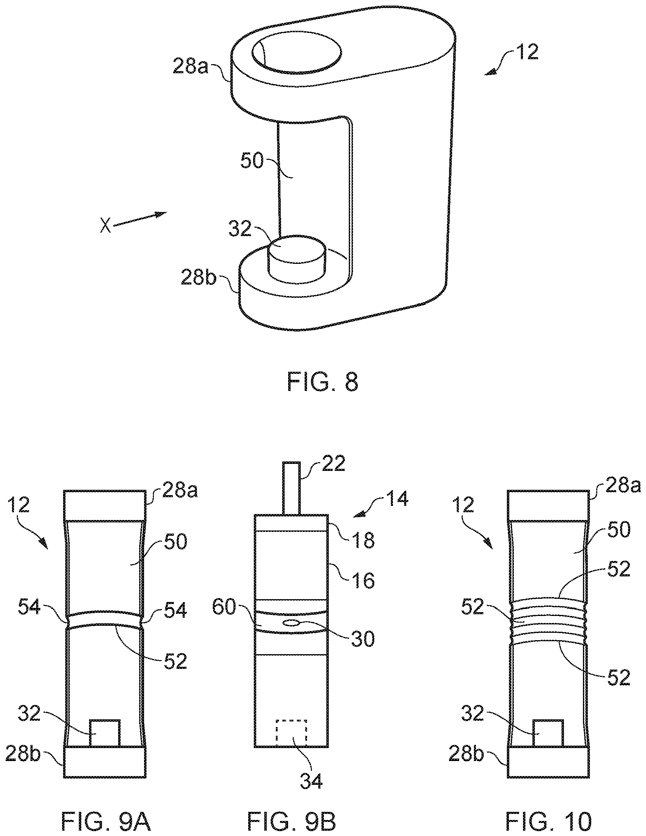

Figure 8 shows a perspective view of an example battery section 12 such as that shown in Figure 1. Supporting sections 28a, 28b including connecting portion 28b extend laterally from the battery section to receive, support and hold an aerosol delivery section, and make an electrical connection to the aerosol delivery section via a connector 32. The battery section 12 has a side wall 50 which will face a side wall of a joined aerosol delivery section. The interface region lies in the area of these side walls in the following embodiments.

Figure 9A shows a side view of a battery section 12 according to an embodiment of the invention, from the direction of arrow X in Figure 8. In this example, the side wall 50 has a groove 52 defined in its surface, extending substantially horizontally across the wall 50 from one side to the other. The groove 52 terminates at the edges of the wall, in this example in the form of notches 54 in the edges. Hence, the groove ends 54, which form the external openings to air in this embodiment, will be externally visible in this example. Alternatively, certain shapes of battery section, particularly those like this embodiment where the side wall 50 is concave, will allow the groove ends 54 to be formed so as to be less visible.

Figure 9B shows a schematic side view of an aerosol delivery section 14 suitable for use with the battery section 12 of Figure 9A. An air inlet 30 is formed in the side wall 60. In this aerosol delivery section, the air inlet 30 is near the base of the tank 16, but it may be disposed elsewhere in the interface region, with corresponding positioning of the groove 54 so that the air inlet 30 and the groove can be aligned. In use, the two sections are joined so that the side wall 60 of the aerosol delivery section faces the side wall 50 of the battery section 12, defining the interface region.

The groove 52, when brought together with the side wall 60 of the aerosol delivery section 14, can be thought of as a cavity of a particular shape, having two external openings to air (one each side of the battery section). A groove with only one external end might be provided instead, but the use of more than one external opening to air reduces the risk of accidental blockage of the air intake. Additionally, grooves of other shapes and configurations, with various numbers of external openings, may be used instead.

Figure 10 shows a battery section 12, according to an embodiment of the invention, in side view, configured with a shaped side wall. In this embodiment, three substantially parallel horizontal grooves are provided, each extending to both edges of the side wall 50 to give a total of six external openings to air. To function with this cavity arrangement, the aerosol delivery section might have three individual air inlets each connected to the airflow path, one for each groove 52, or a single air inlet having a large vertical extent that traverses all three grooves 52.

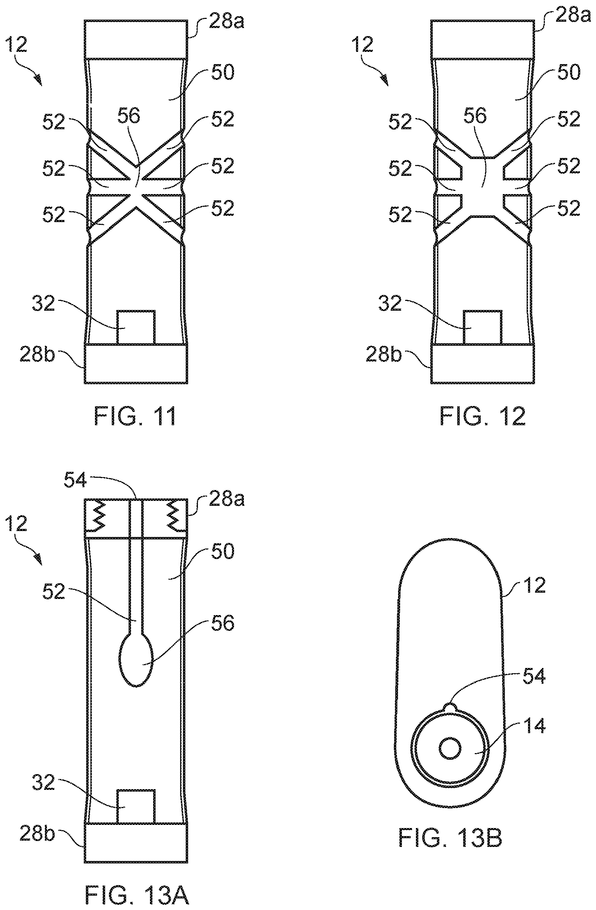

Figure 11 shows another battery section 12, according to an embodiment of the invention, in side view, configured with a shaped side wall. In this example, a total of six grooves 52 are provided to define a cavity, three of which create external openings to air on one edge of the side wall 50 and three of which create external openings to air on the opposite side of the side wall 50. The grooves are angled so as to all meet together at their ends opposite to the external opening ends, forming a star shape. An arrangement such as this, in which multiple external openings connect to a single central recess 56 to which the air inlet will be aligned, provide the advantage of multiple air intake positions while avoiding any need for a large air inlet or multiple air inlets.

Figure 12 shows a similar embodiment of the invention to that of Figure 11. In this embodiment, again shown in side view, the six grooves 52 in the side wall 50 meet at a much larger central recess 56. This provides the advantages of the Figure 11 example together with more flexibility in the positioning of the air inlet for proper alignment with the cavity. This can give improved design freedom, for example, or require a less precise fit or coupling when a user joins the aerosol delivery section to the battery section 12.

Embodiments of this type in which recesses are formed in the battery section side wall are not limited to configurations in which the recesses define external openings to air at the sides of the e-cigarette. The recesses or grooves may alternatively or additionally be arranged to as to define one or more external openings at the top of the device. To achieve this, the recess(es) may be aligned more vertically.

Figure 13A shows a side view of a battery section 12, according to an embodiment of the invention, configured in this way. The upper supporting portion 28a is shown cut away to reveal the top part of the side wall 50. A vertical groove 52 is shaped into the side wall 50 extending from the top edge where it defines an external opening to air 54, down to a larger recess 56 where the air inlet on the aerosol delivery section can be aligned. Figure 13B shows a plan view of the top of the battery section 12, showing how the groove forms the external opening to air 54 at the top edge of the wall when the aerosol delivery system 14 is inserted into the battery section 12.

More than one vertical groove might be provided, which may or may not terminate in a common recess. Also, one or more substantially vertical grooves may be combined with one or more substantially horizontal grooves, so provide external openings to air at both the top and sides of the device. The sides of the device offer more space to accommodate a larger number of air intakes, but since these are more vulnerable to being covered in use than an intake at the top of the device, a top intake can be provided as well to ensure that there is at least one intake that is highly unlikely to be accidentally blocked.

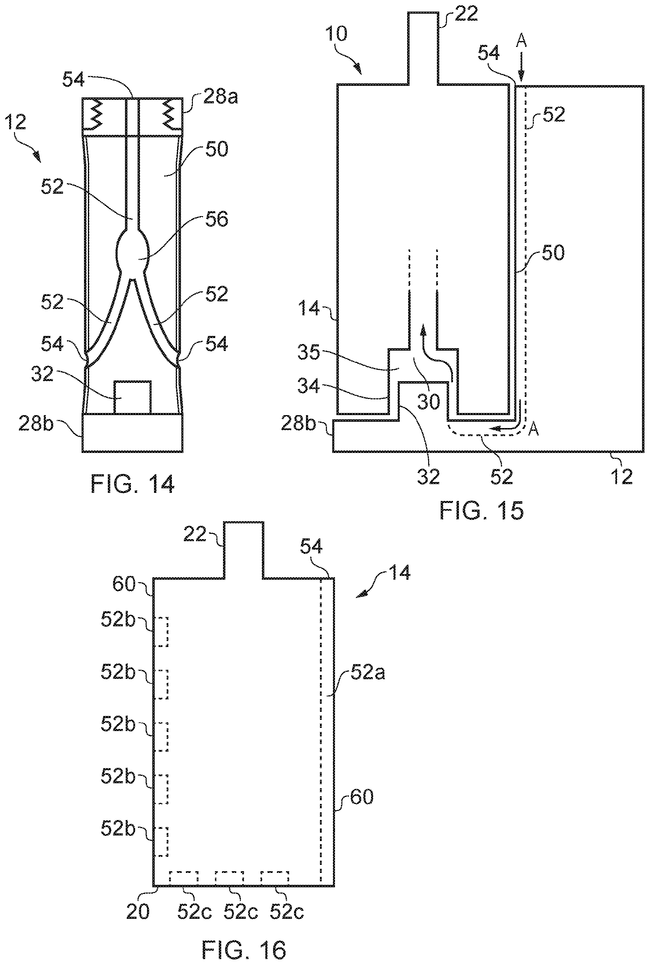

Figure 14 shows a side view of a battery section, according to an embodiment of the invention, configured in this manner. It has the same features as the battery section shown in Figure 13A, but also includes two additional grooves 52 which extend between external openings to air 54 in opposite side edges of the interface region and the larger recess 56. More such grooves or just one such groove might be included if desired.

A further alternative is to combine a groove in the battery section side wall 50 with one or more grooves in the connecting portion such as in the embodiments of Figures 5, 6 and 7. A groove in the battery section side wall may commence at an external opening in an edge of the interface region, and extend to the lower part of the battery section side wall to connect to a groove in the connection portion upper surface, to provide an air pathway to an air inlet in the base wall of the aerosol delivery section.

Figure 15 shows a schematic cross-sectional view of an example vapour provision device 10 having these features. The battery section 12 has formed in its side wall 50 a vertical groove 52 extending from an external opening to air 54 at its top edge adjacent the mouthpiece 22 of the aerosol delivery section 14 down to the bottom edge of the side wall 50, where it connects to a groove 52 in the upper face of the connecting portion 28b. This groove 52 is in airflow communication with a cavity 35 between the connector 32 of the connecting portion 28b and the socket 34 in the base of the aerosol delivery section 14. In this way, air taken in at the external opening 54 at the top of the system flows along the connected grooves 54 to the cavity 35 and into the air inlet 30, to enter the airflow path through the aerosol delivery section 14.

Grooves of any size, shape, position and quantity can be provided to carry air from the edge of the interface region (the exterior junction between the aerosol delivery section and the battery section) to an air inlet located inside the interface region. The grooves need not be straight or of constant width, and may meet together or remain separate. Each groove, or the overall cavity defined by one or more grooves, may have one or more than one external opening to air.

The examples and embodiments discussed above have utilised various shapings in the outer surface of the battery section to achieve the desired channelling of air from the external intake(s) to the interior air inlet on the aerosol delivery section. Equivalent effects can be readily achieved, in comparative examples not falling within the scope of the invention, by using shaping of the outer surface of the aerosol delivery section instead, or combining shaping of the aerosol delivery section and the battery section. Moreover, precise shapings such as those described herein may not be required. Instead, in comparative examples not falling within the scope of the invention, the aerosol delivery section and the battery section may be configured such that when they are joined, the facing surfaces are spaced apart sufficiently to define an air gap in communication with the external air over part or all of the interface region.

Figure 16 shows a highly schematic and simplified side view of an aerosol delivery section, according to a comparative example, with shapings in its outer surface to channel air into an air inlet in an interface region. Note that the air inlet or inlets are not shown in this illustration, but may be positioned where desired to be in airflow communication with air guided by the shapings. Three example positions for possible shapings are shown in phantom; an individual device may have one or some of these only. As a first example, the aerosol delivery section 14 has a vertical groove 52a in a side wall 60 which in use will face a battery section, with an external opening to air 54 at its upper end. This is functionally equivalent to the vertical groove 52 in the battery section shown in Figure 15. One or more vertical grooves or recesses 54a might be formed, which may or may not extend the full height of the aerosol delivery section 14. As a second example, the aerosol delivery section 14 has a series of horizontal grooves 52b formed in a side wall 60 which in use will face a battery section. Any number of such grooves (which may have any shape, such as a corrugation or concertina shape if many grooves are desired) may be included, which may come together to feed a single air intake, or may each feed a separate air intake. A third example is a series of grooves or recesses 54c in the base wall 20 of the aerosol delivery section 54, which will face the upper surface of a connecting portion of a battery section when the two sections are joined. These grooves 54c are in airflow communication with an air inlet in the base wall 20, such as in Figures 3, 5A, 6A and 7A, such as via a cavity 35 as in Figures 5A, 6A or 7A, and might be radially arranged similar to the grooves in the Figure 5B embodiment.

Any of the embodiments and examples presented herein may further comprise an airflow adjuster by which a user can modify the level of airflow in the airflow path and hence control the amount of aerosol delivered per inhalation. Any suitable adjuster may be employed. For example, the adjuster may comprise a movable element such as a curved or flat plate or ring which is slidable over the air inlet so as to partially cover the air inlet and alter an area of the effective bore of the air inlet. The adjuster is preferably configured such that the air inlet cannot be completely covered by the movable element (which would block the airflow path), and may be configured for continuous adjustment or for stepped adjustment between two or more predetermined air inlet sizes and the corresponding air flow levels.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein, as long as the resulting subject-matter falls within the scope of one of the independent claims.

## Claims

1. A vapour provision system (10) comprising:
an aerosol delivery section (14) configured to generate aerosol from liquid in a reservoir (16);
an airflow path (17) through the aerosol delivery section extending from an air inlet (30) to a mouthpiece (22);
a battery section (12) configured to join to the aerosol delivery section and house a battery (26) to provide electrical power to one or more components in the aerosol delivery section, the battery section arranged laterally to at least a portion of the aerosol delivery section with respect to a direction of airflow through the mouthpiece; and
an interface region in which a surface of the aerosol delivery section faces a surface of the battery section when the sections are joined; wherein
the air inlet is located on the aerosol delivery section in the interface region so as to take in air that has been channelled over part of the battery section; and
**characterised in that**: the surface of the battery section in the interface region comprises at least one groove (40; 52) with at least one end (42; 54) located at an edge of the interface region,
such that, when the battery section an the aerosol delivery section are joined:
a first cavity is at least partially formed in the interface region by the at least one groove, the first cavity having at least one external opening to air defined by the at least one end of the at least one groove; and
the at least one external opening to air is in airflow communication with the air inlet via said first cavity.

2. A vapour provision system (10) according to claim 1, in which the battery section (12) is arranged laterally to the aerosol delivery section (14) with respect to a direction of airflow through the mouthpiece (22).

3. A vapour provision system (10) according to claim 1 or claim 2, in which the battery section (12) has a connecting portion (28b) that extends laterally to receive a base part of the aerosol delivery section (14), and the air inlet (30) is located in a base wall (20) of the aerosol delivery section that faces the connecting portion when the sections are joined,
wherein a surface of the connecting portion that faces the base part of the aerosol delivery section has formed therein the at least one groove (40; 52).

4. A vapour provision system (10) according to claim 3, the at least one groove (40; 52) extending radially with respect to a central axis of the aerosol delivery section (14) when joined to the battery section (12) to said at least one end (42; 54) of the at least one groove.

5. A vapour provision system (10) according to claim 4, in which the base part of the aerosol delivery section (14) and the said surface of the connecting portion (28b) are shaped to form a central cavity (35) in the interface region with which each groove (40; 52) is in airflow communication at an end opposite to the external opening end (42; 54), the air inlet (30) being in airflow communication with the central cavity.

6. A vapour provision system (10) according to claim 5, in which the central cavity (35) houses an electrical connection between the battery section (12) and the aerosol delivery section (14).

7. A vapour provision system (10) according to claim 1 or 2, in which said surface of the battery section (12) in the interface region is a side wall (50) of the battery section and the said surface of the aerosol delivery section (14) in the interface region is a side wall of the aerosol delivery section.

8. A vapour provision system (10) according to claim 7, the at least one groove (52) extending across the said side wall (50) of the battery section (12) from a first end (54) to a second end (54), each of the first and second ends forming one of said external openings to air.

9. A vapour provision system (10) according to claim 7, in which each groove (52) has an end (54) forming one of said external openings to air, said end located at an edge of the interface region proximate the mouthpiece (22).

10. A vapour provision system (10) according to any one of claims 1 to 9, in which the first cavity has at least two external openings to air.

11. A battery section (12) for a vapour provision system (10),
the battery section configured to join to an aerosol delivery section (14) of the vapour provision system,
the vapour provision system further comprising an airflow path through the aerosol delivery section extending from an air inlet (30) to a mouthpiece (22),
the battery section configured to house a battery (26) to provide electrical power to said aerosol delivery section when the battery section is joined to said aerosol delivery
the battery section configured to be arranged laterally to at least a portion of the aerosol delivery section with respect to a direction of airflow through the mouthpiece,
the battery section comprising:
an interface region comprising a surface of the battery section configured to face a surface of said aerosol delivery section when the battery section is joined to said aerosol delivery section;
**characterised in that:**
the battery section further comprises at least one groove (40; 52) formed in said surface of the battery section in the interface region positioned for alignment with said air inlet in said aerosol delivery section, the at least one groove having at least one end (42; 54) at an edge of the interface region,
such that, when the battery section and the aerosol delivery section are joined:
a first cavity is at least partially formed in the interface region by the at least one groove, the first cavity having at least one external opening to air defined by the at least one end of the at least one groove; and
the at least one external opening to air is in airflow communication with the air inlet via said first cavity.

## Patentansprüche

1. Dampfbereitstellungssystem (10), umfassend:
einen Aerosolzuführungsabschnitt (14), der dazu ausgelegt ist, aus Flüssigkeit in einem Behälter (16) Aerosol zu erzeugen;
einen Luftstrompfad (17) durch den Aerosolzuführungsabschnitt, der sich von einem Lufteinlass (30) zu einem Mundstück (22) erstreckt;
einen Batterieabschnitt (12), der dazu ausgelegt ist, mit dem Aerosolzuführungsabschnitt zusammengeführt zu werden und eine Batterie (26) zu beinhalten, die einer oder mehreren Komponenten in dem Aerosolzuführungsabschnitt elektrische Energie bereitstellt, wobei der Batterieabschnitt in Bezug auf eine Richtung von Luftstrom durch das Mundstück zu mindestens einer Partie des Aerosolzuführungsabschnittes lateral angeordnet ist; und
eine Schnittstellenregion, in der eine Oberfläche des Aerosolzuführungsabschnittes einer Oberfläche des Batterieabschnitts zugewandt ist, wenn die Abschnitte zusammengeführt werden; wobei
sich der Lufteinlass auf dem Aerosolzuführungsabschnitt in der Schnittstellenregion befindet, um Luft, die über einen Teil des Batterieabschnittes kanalisiert wurde, aufzunehmen; und
**gekennzeichnet dadurch, dass**
die Oberfläche des Batterieabschnittes in der Schnittstellenregion mindestens eine Nut (40; 52) mit mindestens einem Ende (42; 54) umfasst, befindlich an einer Kante der Schnittstellenregion,
sodass dann, wenn der Batterieabschnitt und der Aerosolzuführungsabschnitt zusammengeführt sind:
ein erster Hohlraum durch die mindestens eine Nut in der Schnittstellenregion mindestens teilweise ausgebildet ist, wobei der erste Hohlraum mindestens eine externe Öffnung zu Luft aufweist, die durch das mindestens eine Ende der mindestens einen Nut definiert ist; und
die mindestens eine externe Öffnung zu Luft über den ersten Hohlraum in einem Luftstromaustausch mit dem Lufteinlass steht.

2. Dampfbereitstellungssystem (10) nach Anspruch 1, in dem der Batterieabschnitt (12) mit Bezug auf eine Richtung von Luftstrom durch das Mundstück (22) lateral zu dem Aerosolzuführungsabschnitt (14) angeordnet ist.

3. Dampfbereitstellungssystem (10) nach Anspruch 1 oder Anspruch 2, in dem der Batterieabschnitt (12) einen verbindenden Abschnitt (28b) aufweist, der sich lateral erstreckt, um ein Basisteil des Aerosolzuführungsabschnitts (14) aufzunehmen, und der Lufteinlass (30) befindet sich in einer Basiswand (20) des Aerosolzuführungsabschnitts, die dem verbindenden Abschnitt zugewandt ist, wenn die Abschnitte zusammengeführt sind, wobei in einer Oberfläche des verbindenden Abschnitts, die dem Basisteil des Aerosolzuführungsabschnitts zugewandt ist, die mindestens eine Nut (40; 52) ausgebildet ist.

4. Dampfbereitstellungssystem (10) nach Anspruch 3, wobei sich die mindestens eine Nut (40; 52) in Bezug auf eine mittlere Achse des Aerosolzuführungsabschnitts (14), wenn mit dem Batterieabschnitt (12) zusammengeführt, radial zu dem mindestens einen Ende (42; 54) der mindestens einen Nut erstreckt.

5. Dampfbereitstellungssystem (10) nach Anspruch 4, in dem das Basisteil des Aerosolzuführungsabschnitts (14) und die Oberfläche des verbindenden Abschnitts (28b) so geformt sind, dass sie in der Schnittstellenregion einen zentralen Hohlraum (35) ausbilden, mit dem sich jede Nut (40; 52) an einem dem externen Öffnungsende (42; 54) gegenüberliegenden Ende in einem Luftstromaustausch befindet, wobei sich der Lufteinlass (30) in einem Luftstromaustausch mit dem zentralen Hohlraum befindet.

6. Dampfbereitstellungssystem (10) nach Anspruch 5, wobei in dem zentralen Hohlraum (35) eine elektrische Verbindung zwischen dem Batterieabschnitt (12) und dem Aerosolzuführungsabschnitt (14) untergebracht ist.

7. Dampfbereitstellungssystem (10) nach Anspruch 1 oder 2, in dem die seitliche Oberfläche des Batterieabschnitts (12) in der Schnittstellenregion eine seitliche Wand (50) des Batterieabschnitts ist und die seitliche Oberfläche des Aerosolzuführungsabschnitts (14) in der Schnittstellenregion eine Seitenwand des Aerosolzuführungsabschnitts ist.

8. Dampfbereitstellungssystem (10) nach Anspruch 7, wobei sich die mindestens eine Nut (52) über die seitliche Wand (50) des Batterieabschnitts (12) von einem ersten Ende (54) zu einem zweiten Ende (54) erstreckt, wobei jedes von dem ersten und zweiten Ende eine der externen Öffnungen zu Luft ausbildet.

9. Dampfbereitstellungssystem (10) nach Anspruch 7, in dem jede Nut (52) ein Ende (54) aufweist, das eine der externen Öffnungen zu Luft ausbildet, wobei sich das Ende an einer Kante der Schnittstellenregion in der Nähe des Mundstücks (22) befindet.

10. Dampfbereitstellungssystem (10) nach einem der Ansprüche 1 bis 9, in dem der erste Hohlraum mindestens zwei externe Öffnungen zu Luft aufweist.

11. Batterieabschnitt (12) für ein Dampfbereitstellungssystem (10), wobei der Batterieabschnitt dazu ausgelegt ist, mit einem Aerosolzuführungsabschnitt (14) des Dampfbereitstellungssystems zusammengeführt zu werden, wobei das Dampfbereitstellungssystem ferner einen Luftstrompfad durch den Aerosolzuführungsabschnitt umfasst, der sich von einem Lufteinlass (30) zu einem Mundstück (22) erstreckt;
wobei der Batterieabschnitt dazu ausgelegt ist, eine Batterie (26) zu beinhalten, die dem Aerosolzuführungsabschnitt elektrische Energie bereitstellt, wenn der Batterieabschnitt mit dem Aerosolzuführungsabschnitt zusammengeführt ist, dazu ausgelegt, in Bezug auf eine Richtung von Luftstrom durch das Mundstück zu mindestens einer Partie des Aerosolzuführungsabschnittes lateral angeordnet zu sein,
wobei der Batterieabschnitt Folgendes umfasst:
eine Schnittstellenregion, die eine Oberfläche des Batterieabschnittes umfasst, die dazu ausgelegt ist, einer Oberfläche des Aerosolzuführungsabschnittes zugewandt zu sein, wenn der Batterieabschnitt mit dem Aerosolzuführungsabschnitt zusammengeführt ist;
**dadurch gekennzeichnet, dass**:
der Batterieabschnitt ferner mindestens eine Nut (40; 52) umfasst, die in der Oberfläche des Batterieabschnitts in der Schnittstellenregion ausgebildet ist, positioniert zur Ausrichtung auf den Lufteinlass in dem Aerosolzuführungsabschnitt, wobei die mindestens eine Nut mindestens ein Ende (42; 54) an einer Kante der Schnittstellenregion aufweist, sodass dann, wenn der Batterieabschnitt und der Aerosolzuführungsabschnitt zusammengeführt sind:
ein erster Hohlraum durch die mindestens eine Nut in der Schnittstellenregion mindestens teilweise ausgebildet ist, wobei der erste Hohlraum mindestens eine externe Öffnung zu Luft aufweist, die durch das mindestens eine Ende der mindestens einen Nut definiert ist; und
die mindestens eine externe Öffnung zu Luft über den ersten Hohlraum in einem Luftstromaustausch mit dem Lufteinlass steht.

## Revendications

1. Système de production de vapeur (10) comprenant :
une section d'administration d'aérosol (14) conçue pour générer un aérosol à partir d'un liquide dans un réservoir (16) ;
un trajet d'écoulement d'air (17) à travers la section d'administration d'aérosol s'étendant depuis une admission d'air (30) vers un embout buccal (22) ;
une section batterie (12) conçue pour se joindre à la section d'administration d'aérosol et pour accueillir une batterie (26) pour alimenter électriquement un ou plusieurs éléments de la section d'administration d'aérosol, la section batterie étant agencée latéralement à au moins une partie de la section d'administration d'aérosol par rapport à une direction d'un écoulement d'air à travers l'embout buccal ; et
une région d'interface dans laquelle une surface de la section d'administration d'aérosol est tournée vers une surface de la section batterie lorsque les sections sont jointes ; dans lequel
l'admission d'air est située sur la section d'administration d'aérosol dans la région d'interface de façon à faire entrer l'air qui a été canalisé sur une partie de la section batterie ; et
**caractérisé en ce que** :
la surface de la section batterie dans la région d'interface comprend au moins une rainure (40 ; 52) possédant au moins une extrémité (42 ; 54) située au niveau d'un bord de la région d'interface, de sorte que, lorsque la section batterie et la section d'administration d'aérosol sont jointes :
une première cavité soit au moins partiellement formée dans la région d'interface par l'au moins une rainure, la première cavité possédant au moins une ouverture externe donnant sur l'air définie par l'au moins une extrémité de l'au moins une rainure ; et que
l'au moins une ouverture externe vers l'air soit en communication d'écoulement d'air avec l'admission d'air par l'intermédiaire de ladite première cavité.

2. Système de production de vapeur (10) selon la revendication 1, dans lequel la section batterie (12) est agencée latéralement à la section d'administration d'aérosol (14) par rapport à une direction d'écoulement d'air à travers l'embout buccal (22).

3. Système de production de vapeur (10) selon la revendication 1 ou revendication 2, dans lequel la section batterie (12) possède une partie raccordement (28b) qui s'étend latéralement pour accueillir une partie de base de la section d'administration d'aérosol (14), et l'admission d'air (30) est située dans une paroi de base (20) de la section d'admission d'aérosol qui est tournée vers la partie raccordement lorsque les sections sont jointes,
dans lequel une surface de la partie raccordement qui est tournée vers la partie de base de la section d'administration d'aérosol possède, formée en son sein, l'au moins une rainure (40 ; 52).

4. Système de production de vapeur (10) selon la revendication 3, l'au moins une rainure (40 ; 52) s'étendant radialement par rapport à un axe central de la section d'administration d'aérosol (14) lorsqu'elle est jointe à la section batterie (12) vers ladite au moins une extrémité (42 ; 54) de l'au moins une rainure.

5. Système de production de vapeur (10) selon la revendication 4, dans lequel la partie de base de la section d'administration d'aérosol (14) et ladite surface de la partie raccordement (28b) sont façonnées pour former une cavité centrale (35) dans la région d'interface avec laquelle chaque rainure (40 ; 52) est en communication d'écoulement d'air au niveau d'une extrémité opposée à l'extrémité d'ouverture externe (42 ; 54), l'admission d'air (30) étant en communication d'écoulement d'air avec la cavité centrale.

6. Système de production de vapeur (10) selon la revendication 5, dans laquelle la cavité centrale (35) accueille une connexion électrique entre la section batterie (12) et la section d'administration d'aérosol (14).

7. Système de production de vapeur (10) selon la revendication 1 ou 2, dans lequel ladite surface de la section batterie (12) dans la région d'interface est une paroi latérale (50) de la section batterie et ladite surface de la section d'administration d'aérosol (14) dans la région d'interface est une paroi latérale de la section d'administration d'aérosol.

8. Système de production de vapeur (10) selon la revendication 7, l'au moins une rainure (52) s'étendant en travers de ladite paroi latérale (50) de la section batterie (12) depuis une première extrémité (54) vers une seconde extrémité (54), chacune des première et seconde extrémités formant une desdites ouvertures externes donnant sur l'air.

9. Système de production de vapeur (10) selon la revendication 7, dans lequel chaque rainure (52) possède une extrémité (54) formant une desdites ouvertures externes donnant sur l'air, ladite extrémité étant située au niveau d'un bord de la région d'interface à proximité de l'embout buccal (22).

10. Système de production de vapeur (10) selon l'une quelconque des revendications 1 à 9, dans lequel la première cavité possède au moins deux ouvertures externes donnant sur l'air.

11. Section batterie (12) pour un système de production de vapeur (10),
la section batterie étant conçue pour se joindre à une section d'administration d'aérosol (14) du système de production de vapeur,
le système de production de vapeur comprenant en outre un trajet d'écoulement d'air à travers la section d'administration d'aérosol s'étendant depuis une admission d'air (30) vers un embout buccal (22),
la section batterie étant conçue pour accueillir une batterie (26) pour alimenter électriquement ladite section d'administration d'aérosol lorsque la section batterie est jointe à ladite administration d'aérosol la section batterie étant conçue pour être agencée latéralement à au moins une partie de la section d'administration d'aérosol par rapport à une direction d'un écoulement d'air à travers l'embout buccal,
la section batterie comprenant :
une région d'interface comprenant une surface de la section batterie conçue pour être tournée vers une surface de ladite section d'administration d'aérosol lorsque la section batterie est jointe à ladite section d'administration d'aérosol ;
**caractérisé en ce que** :
la section batterie comprend en outre au moins une rainure (40 ; 52) formée dans ladite surface de la section batterie dans la région d'interface positionnée pour un alignement sur ladite admission d'air dans ladite section d'admission d'aérosol, l'au moins une rainure possédant au moins une extrémité (42 ; 54) au niveau d'un bord de la région d'interface,
de sorte que, lorsque la section batterie et la section d'administration d'aérosol sont jointes :
une première cavité soit au moins partiellement formée dans la région d'interface par l'au moins une rainure, la première cavité possédant au moins une ouverture externe donnant sur l'air définie par l'au moins une extrémité de l'au moins une rainure ; et que l'au moins une ouverture externe donnant sur l'air soit en communication d'écoulement d'air avec l'admission d'air par l'intermédiaire de ladite première cavité.
